(19) Europäisches Patentamt
European Patent Office
Office européen des brevets

(11) Veröffentlichungsnummer: **0 033 160**
**B1**

(12) # EUROPÄISCHE PATENTSCHRIFT

(45) Veröffentlichungstag der Patentschrift: **02.05.85**

(21) Anmeldenummer: **81100615.4**

(22) Anmeldetag: **28.01.81**

(51) Int. Cl.⁴: **C 07 C 121/75,** C 07 C 69/74, C 07 C 61/04, C 07 C 67/10, A 01 N 53/00

(54) **Substituierte 3-(1,2-Dibrom-alkyl)-2,2-dimethyl-cyclo-propan-1-carbonsäureester, Verfahren sowie Zwischenprodukte zu ihrer Herstellung und ihre Verwendung in Schädlingsbekämpfungsmitteln.**

(30) Priorität: **05.02.80 DE 3004092**

(43) Veröffentlichungstag der Anmeldung: **05.08.81 Patentblatt 81/31**

(45) Bekanntmachung des Hinweises auf die Patenterteilung: **02.05.85 Patentblatt 85/18**

(84) Benannte Vertragsstaaten: **AT BE CH DE FR GB IT LI NL**

(56) Entgegenhaltungen:
**EP - A - 0 001 795**
**EP - A - 0 001 944**
**EP - A - 0 006 600**
**EP - A - 0 015 239**
**FR - A - 2 364 884**
**FR - A - 2 380 246**
**FR - A - 2 380 247**
**FR - A - 2 380 248**
**FR - A - 2 398 041**
**FR - A - 2 398 457**
**FR - A - 2 419 932**
**FR - A - 2 428 396**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Fuchs, Rainer, Dr., Roeberstrasse 8, D-5600 Wuppertal 1 (DE)**
Erfinder: **Naumann, Klaus, Dr., Richard-Wagner-Strasse 9, D-5090 Leverkusen (DE)**
Erfinder: **Hammann, Ingeborg, Dr., Belfortstrasse 9, D-5000 Köln (DE)**
Erfinder: **Stendel, Wilhelm, Dr., In den Birken 55, D-5600 Wuppertal 1 (DE)**

**EP 0 033 160 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft substituierte 3-(1,2-Dibromalkyl)-2,2-dimethyl-cyclopropan-1-carbonsäureester, Verfahren zu ihrer Herstellung, ihre Verwendung in Schädlingsbekämpfungsmitteln, insbesondere in Insektiziden und Akariziden, sowie neue Zwischenprodukte zu ihrer Herstellung.

Es ist bekannt, dass bestimmte Cyclopropancarbonsäureester, wie z.B. 3-(2,2-Dichlorvinyl)-2,2-dimethyl-cyclopropan-1-carbonsäure-3-phenoxy-benzylester und -3-phenoxy-α-cyanobenzylester insektizide und akarizide Eigenschaften aufweisen (vergleiche US-PS 4 024 163 und 4 031 239). Die Wirkung dieser Verbindungen ist jedoch, insbesondere bei niedrigen Wirkstoffkonzentrationen und Aufwandmengen, nicht immer zufriedenstellend.

Gegenstand der vorliegenden Erfindung sind:

1. Substituierte 3-(1,2-Dibrom-alkyl)-2,2-dimethylcyclopropan-1-carbonsäureester der Formel I

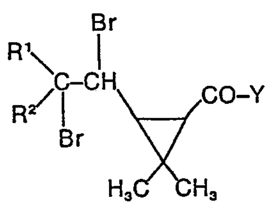

(I)

in welcher

$R^1$ für Halogen oder für gegebenenfalls substituiertes Phenyl oder Alkyl steht,

$R^2$ für Wasserstoff, Halogen oder gegebenenfalls substituiertes Alkyl steht und

Y für den Rest

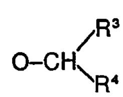

steht, wobei

$R^3$ für Wasserstoff, Cyano oder für gegebenenfalls substituiertes Alkyl, Alkenyl oder Alkinyl steht, und

$R^4$ für Phenyl steht, welches durch Halogen und/oder durch gegebenenfalls durch Halogen, Alkyl oder Halogenalkyl substituiertes Phenoxy substituiert ist mit der Massgabe, dass der Phenylrest wenigstens einen Halogen-Substituenten enthält, wenn $R^1$ für Methyl oder Halogen steht.

2. Verfahren zur Herstellung von Verbindungen der Formel (I), dadurch gekennzeichnet, dass man

a) 3-Alkenyl-2,2-dimethyl-cyclopropan-1-carbonsäureester der Formel (II)

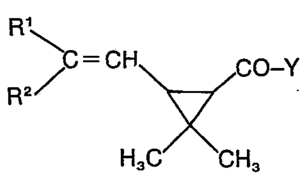

(II)

in welcher

$R^1$, $R^2$ und Y die oben angegebenen Bedeutungen haben, mit Brom, gegebenenfalls in Gegenwart eines Verdünnungsmittels umsetzt, oder

b) 3-(1,2-Dibrom-alkyl)-2,2-dimethyl-cyclopropan-1-carbonsäuren der Formel III

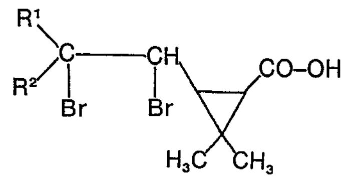

(III)

in welcher

$R^1$ und $R^2$ die oben angegebenen Bedeutungen haben, oder reaktionsfähige Derivate derselben mit einem bei Pyrethroiden üblichen Alkohol der Formel IV

Y–H (IV)

in welcher

Y die oben angegebene Bedeutung hat, oder mit reaktionsfähigen Derivaten desselben, gegebenenfalls in Gegenwart von Säurebindemitteln, gegebenenfalls in Gegenwart von Katalysatoren und gegebenenfalls in Gegenwart von Verdünnungsmitteln umsetzt.

3. 3-(1,2-Dibrom-alkyl)-2,2-dimethyl-cyclopropan-1-carbonsäure-Derivate der Formel III a

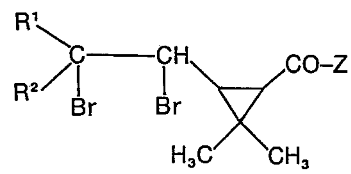

(III a)

in welcher

$R^2$ für Wasserstoff, Halogen oder gegebenenfalls substituiertes Alkyl steht,

$R^1$ für gegebenenfalls substituiertes Phenyl steht und

Z für Hydroxy, Chlor, $C_1$–$C_4$-Alkoxy oder für $O^{\ominus}M^{\oplus}$ steht, wobei $M^{\oplus}$ für ein Ammoniumion oder ein Alkali- oder Erdalkalimetallionenäquivalent steht.

4. Verfahren zur Herstellung von Verbindungen der Formel (III a), dadurch gekennzeichnet, dass man 3-Alkenyl-2,2-dimethyl-cyclopropan-1-carbonsäuren der Formel V

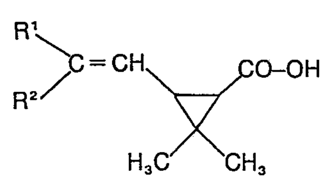

(V)

in welcher

$R^1$ und $R^2$ die oben angegebenen Bedeutungen haben, mit Brom, gegebenenfalls in Gegenwart eines Verdünnungsmittels umsetzt und die Bromierungsprodukte gegebenenfalls nach üblichen Methoden in die entsprechenden Carbonsäurederivate nach Formel (IIIa) umwandelt.

Die substituierten 3-(1,2-Dibrom-alkyl)-2,2-dimethyl-cyclopropan-1-carbonsäureester zeichnen sich durch hohe insektizide und akarizide Wirksamkeit aus.

Überraschenderweise zeigen die erfindungsgemässen Verbindungen der Formel (I) eine erheblich höhere insektizide und akarizide Wirkung als aus dem Stand der Technik bekannte Verbindungen analoger Konstitution und gleicher Wirkungsrichtung.

Gegenstand der Erfindung sind vorzugsweise Verbindungen der Formel (I), in welcher

$R^1$ für gegebenenfalls durch Halogen und/oder gegebenenfalls halogen-substituierte Reste $C_1$–$C_4$-Alkyl, $C_1$–$C_4$-Alkoxy, $C_1$–$C_4$-Alkylthio oder $C_1$–$C_2$-Alkylendioxy substituiertes Phenyl, für gegebenenfalls halogen-substituiertes $C_1$–$C_4$-Alkyl oder für Halogen steht,

$R^2$ für Wasserstoff, Halogen oder für gegebenenfalls halogen-substituiertes $C_1$–$C_4$-Alkyl steht, und

Y für den Rest 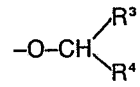

steht, wobei

$R^3$ für Wasserstoff, Cyano oder für gegebenenfalls substituiertes Alkyl, Alkenyl oder Alkinyl steht, und

$R^4$ für Phenyl steht, welches durch Halogen und/oder durch gegebenenfalls durch Halogen, Alkyl oder Halogenalkyl substituiertes Phenoxy substituiert ist mit der Massgabe, dass der Phenylrest wenigstens einen Halogen-Substituenten enthält, wenn $R^1$ für Methyl oder Halogen steht, besonders bevorzugt stehen dabei

$R^3$ für Wasserstoff oder Cyano und

$R^4$ für Pentafluorphenyl, Pentachlorphenyl, 4-Fluor-3-phenoxy-phenyl oder 4-Fluor-3-(4-fluorphenoxy)-phenyl und, für den Fall, dass $R^1$ für gegebenenfalls substituiertes Phenyl steht, auch für 3-Phenoxyphenyl und 3-(4-Fluor-phenoxy)-phenyl.

Verwendet man bei dem unter (2 a) dargelegten Verfahren zur Herstellung von Verbindungen der Formel (I) beispielsweise

3-(2,2-Dibrom-vinyl)-2,2-dimethyl-cyclopropan-1-carbonsäure-4-fluor-3-phenoxy-benzylester

als Ausgangsstoff, so kann die Bromierungsreaktion durch folgendes Formelschema skizziert werden:

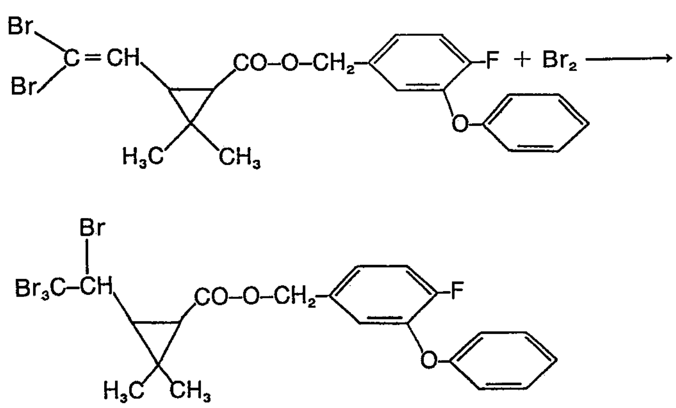

Die als Ausgangsstoffe für Verfahren (2 a) zu verwendenden 3-Alkenyl-2,2-dimethyl-cyclopropan-1-carbonsäurebenzylester sind durch Formel (II) definiert. Vorzugsweise stehen darin $R^1$, $R^2$ und Y für diejenigen Reste, welche bei der Definition der Reste $R^1$, $R^2$ und Y in Formel (I) als bevorzugt genannt wurden.

Als Beispiele für die Ausgangsstoffe der Formel (II) seien genannt:

3-(2,2-Difluor-vinyl)-, 3-(2,2-Dichlor-vinyl)-,
3-(2,2-Dibrom-vinyl)-, 3-(2-Phenyl-vinyl)-,
3-(2-Chlor-2-phenyl-vinyl)- und
3-(2-Chlor-2-(4-chlor-phenyl)-vinyl)-,
3-(2-Brom-2-(4-chlor-phenyl)-vinyl)-
2,2-dimethyl-cyclopropan-1-carbonsäure-
4-fluor-3-phenoxy-benzylester und
-4-fluor-3-phenoxy-α-cyanobenzylester.

Verbindungen der Formel (II) sind bereits bekannt (vergleiche DE-OS 2 709 264 und 2 730 515 und Chem. Soc. Review Band 7/4 (1978) S. 473 ff).

Verfahren (2 a) wird vorzugsweise unter Verwendung von Verdünnungsmitteln durchgeführt. Als solche kommen praktisch alle gegen Brom inerten organischen Lösungsmittel in Frage. Hierzu gehören insbesondere die aliphatischen halogenierten Kohlenwasserstoffe, wie z.B. Methylenchlorid, Chloroform, Tetrachlorkohlenstoff, und 1,2-Dichlorethan.

Die Reaktionstemperatur wird bei Verfahren (2 a) zwischen −20 und +80 °C, vorzugsweise zwischen 0 und 50 °C gehalten. Das Verfahren wird im allgemeinen bei Normaldruck durchgeführt.

Zur Durchführung des erfindungsgemässen Verfahrens (2 a) werden die Ausgangsstoffe gewöhnlich in äquimolaren Mengen eingesetzt. Ein Überschuss der einen oder anderen Reaktionskomponente bringt keine wesentlichen Vorteile. Die Umsetzung wird im allgemeinen in einem geeigneten Verdünnungsmittel durchgeführt und das Reaktionsgemisch wird mehrere Stunden bei der erforderlichen Temperatur gerührt. Die Reaktionslösung wird anschliessend mit Wasser gewaschen, dann wird von der organischen Phase das Lösungsmittel unter vermindertem Druck bei mässig erhöhter Temperatur sorgfältig abdestilliert («Andestillieren»), wobei man das Produkt im allgemeinen als öligen Rückstand erhält.

Verwendet man bei dem unter (2 b) dargelegten Verfahren zur Herstellung von Verbindungen der Formel (I) beispielsweise
3-(1,2-Dibrom-2-chlor-2-phenyl-ethyl)-
 2,2-dimethyl-cyclopropan-1-carbonsäurechlorid
und 3-Phenoxy-α-cyano-benzylakohol als Ausgangsstoffe, so kann die Reaktion dieser Verbindungen durch folgendes Formelschema skizziert werden:

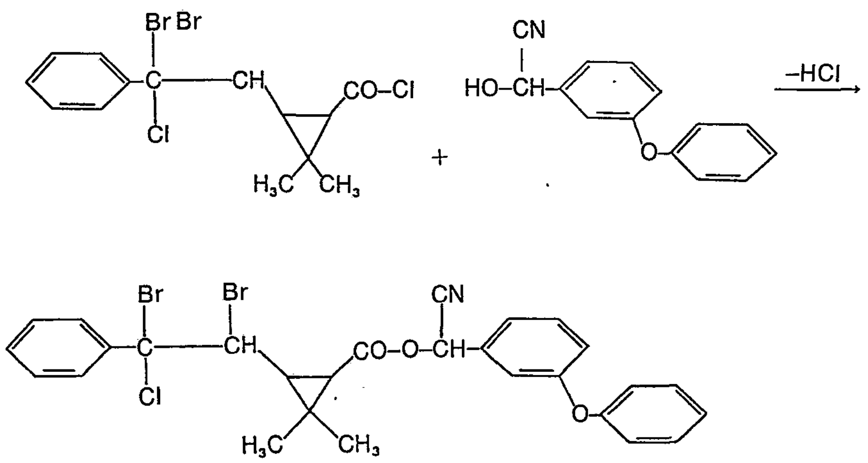


Die als Ausgangsstoffe für Verfahren (2 b) zu verwendenden 3-(1,2-Dibromalkyl)-2,2-dimethyl-cyclopropan-1-carbonsäuren sind durch Formel (III) definiert.

Vorzugsweise stehen darin $R^1$ und $R^2$ für diejenigen Reste, welche bei der Definition der Reste $R^1$ und $R^2$ in Formel (I) als bevorzugt genannt wurden. Als Ausgangsstoffe sind insbesondere die neuen Verbindungen der Formel (III a) bevorzugt, in welcher vorzugsweise

$R^2$ für Wasserstoff, Halogen oder gegebenenfalls halogen-substituiertes Alkyl steht,

$R^1$ für gegebenenfalls durch Halogen und/oder durch gegebenenfalls halogen-substituierte Reste aus der Reihe $C_1$–$C_4$-Alkyl, $C_1$–$C_4$-Alkoxy, $C_1$–$C_4$-Alkylthio und $C_1$–$C_2$-Alkylendioxy substituiertes Phenyl steht, und

Z für Hydroxy, Chlor, $C_1$–$C_4$-Alkoxy oder für $O^{\ominus}M^{\oplus}$ steht, wobei $M^{\oplus}$ für ein Ammoniumion oder ein Alkali- oder Erdalkalimetallionenäquivalent steht.

Als Beispiele für die Ausgangsstoffe der Formeln (III) und/oder (III a) seien genannt:
3-(1,2-Dibrom-2-phenyl-ethyl)-,
3-(1,2-Dibrom-2-(4-chlor-phenyl)-ethyl)-,
3-(1,2-Dibrom-2-chlor-2-phenyl-ethyl)-,
3-(1,2-Dibrom-2-chlor-2-(4-chlor-phenyl)-ethyl)-,
3-(1,2-Dibrom-2-chlor-2-(4-fluor-phenyl)-
 ethyl)- und
3-(1,2-Dibrom-2-chlor-2-(4-trifluor-
 methoxy-phenyl)-ethyl)- und
3-(1,2,2-Tribrom-2-(4-chlor-phenyl)-ethyl)-
 2,2-dimethyl-cyclopropan-1-carbonsäure
sowie die entsprechenden Säurechloride, Methylester und Ethylester.

Man erhält die neuen Carbonsäuren der Formel (III a) nach dem oben unter (4) dargelegten Verfahren, indem man 3-Alkenyl-2,2-dimethyl-cyclopropan-1-carbonsäuren der Formel V (oben), in welcher vorzugsweise

$R^2$ für Wasserstoff, Halogen oder gegebenenfalls halogen-substituiertes Alkyl steht und

$R^1$ für gegebenenfalls durch Halogen und/oder durch gegebenenfalls halogen-substituierte Reste aus der Reihe $C_1$–$C_4$-Alkyl, $C_1$–$C_4$-Alkoxy, $C_1$–$C_4$-Alkylthio und $C_1$–$C_2$-Alkylendioxy substituiertes Phenyl steht,

mit Brom, vorzugsweise in einem inerten Lösungsmittel, wie z.B. Tetrachlorkohlenstoff, bei Temperaturen zwischen 0 und 50 °C umsetzt.

Aus den so erhaltenen Säuren der Formel (III a) können die entsprechenden Säurechloride nach üblichen Methoden, beispielsweise durch Umsetzung mit Thionylchlorid, bei Temperaturen zwischen 10 und 100 °C, gegebenenfalls unter Verwendung eines Verdünnungsmittels, wie z.B. Tetrachlorkohlenstoff, hergestellt werden.

Ebenfalls nach üblichen Methoden erhält man aus den Säurechloriden der Formel (III a) die Ester, beispielsweise durch Umsetzung mit Alkoholen, wie z.B. Methanol oder Ethanol, gegebenenfalls in Gegenwart von Säurebindemitteln, wie

z. B. Pyridin, bei Temperaturen zwischen 10 und 50 °C. Die Salze der Formel (III a) erhält man aus den entsprechenden Säuren durch Umsetzung mit Ammoniak bzw. Aminen oder Alkali- oder Erdalkalihydroxiden, wie z. B. Kaliumhydroxid, gegebenenfalls unter Verwendung von Verdünnungsmitteln, wie z. B. Wasser, Methanol oder Ethanol, bei Temperaturen zwischen 10 und 50 °C.

Die Vorprodukte der Formel (V) sind bekannte Verbindungen (vergleiche DE-OS 2 730 515 und US-PS 4 157 447).

Die bei Verfahren (2 b) weiter als Ausgangsstoffe zu verwendenden Alkohole sind durch Formel (IV) definiert. Vorzugsweise steht darin Y für diejenigen Reste, welche bei der Definition von Y in Formel (I) als bevorzugt genannt wurden.

Als Beispiele für die Ausgangsstoffe der Formel (IV) seien genannt:
3-Phenoxy-benzylakohol,
α-Cyano-3-phenoxy-benzylalkohol,
4-Fluor-3-phenoxy-benzylalkohol,
4-Fluor-3-phenoxy-α-cyano-benzylalkohol,
3-(4-Fluor-phenoxy)-benzylalkohol und
3-(4-Fluor-phenoxy)-α-cyano-benzylalkohol.

Die Ausgangsverbindungen der Formel (IV) sind bekannt (vergleiche DE-OS 2 621 433 und 2 709 264 und Chem. Soc. Review Band 7/4 (1978) S. 473 ff).

Verfahren (2 b) wird vorzugsweise unter Verwendung von Verdünnungsmitteln durchgeführt. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z. B. Acetonitril und Propionitril, Amide, wie z. B. Dimethylformamid, Dimethylacetamid und N-Methylpyrrolidon, sowie Dimethylsulfoxid, Tetramethylensulfon und Hexamethylphosphorsäuretriamid.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Die Reaktionstemperatur kann innerhalb eines grösseren Bereichs variiert werden. Im allgemeinen arbeitet man zwischen 0 und 100 °C, vorzugsweise bei 10 bis 50 °C.

Verfahren (2 b) wird im allgemeinen bei Normaldruck durchgeführt.

Zur Durchführung des erfindungsgemässen Verfahrens werden die Ausgangsstoffe gewöhnlich in äquimolaren Mengen eingesetzt. Ein Überschuss der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt und das Reaktionsgemisch wird mehrere Stunden bei der erforderlichen Temperatur gerührt. Danach giesst man das Reaktionsgemisch in Wasser, trennt die organische Phase, gegebenenfalls nach Zugabe eines mit Wasser nicht mischbaren Lösungsmittels, wie z. B. Toluol, und Durchschütteln ab, wäscht mit Wasser, trocknet, filtriert und destilliert vom Filtrat das Lösungsmittel unter vermindertem Druck ab.

Die neuen Verbindungen fallen in Form von Ölen an, die sich zum Teil nicht unzersetzt destillieren lassen, jedoch durch sogenanntes «Andestillieren», d. h. durch längeres Erhitzen unter vermindertem Druck auf mässig erhöhte Temperaturen von den letzten flüchtigen Anteilen befreit und auf diese Weise gereinigt werden. Zu ihrer Charakterisierung dient der Brechungsindex oder das $^1$HNMR-Spektrum.

Als weitere mögliche, hier nicht näher erläuterte Herstellungsmethode 11 für Verbindungen der Formel (I) sind neben den oben dargelegten Verfahren (2 a) und (2 b) die Umsetzung von Niederalkylestern von Carbonsäuren der Formel (III) mit Alkoholen der Formel (IV), die Umsetzung von Salzen von Carbonsäuren der Formel (III) mit Benzylhalogeniden als reaktionsfähigen Derivaten von Alkoholen der Formel (IV), sowie die Umsetzung von Chloriden von Carbonsäuren der Formel (III) mit Benzaldehyden, welche sich von Benzylalkoholen der Formel (IV) ableiten, in Gegenwart von Alkalicyaniden zu nennen.

Herstellungsbeispiele

Beispiel 1:

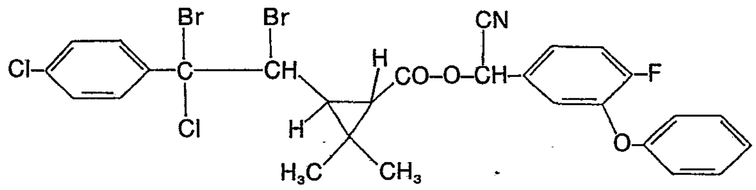

5,1 g (0,01 Mol) trans-3-(E,Z-2-Chlor-2-(4-chlorphenyl)-vinyl)-2,2-dimethyl-cyclopropan-1-(R,S)-carbonsäure-α-(R,S)-cyano-3-phenoxy-4-fluorbenzylester werden in 50 ml Tetrachlorkohlenstoff gelöst und bei 20–25 °C 1,6 g (0,01 Mol) Brom, gelöst in 10 ml Tetrachlorkohlenstoff, unter Rühren zugetropft. Anschliessend wird 12 Stunden bei Raumtemperatur nachgerührt, dann wird das Reaktionsgemisch einmal mit 100 ml Wasser ausgeschüttelt, die organische Phase abgetrennt und das Lösungsmittel im Wasserstrahlvakuum abgezogen. Der Rückstand wird durch kurzes Andestillieren bei 60 °C/2 mbar von letzten Lösungsmittelresten befreit. Man erhält 5,9 g (88% der Theorie) trans-3-(2-Chlor-2-(4-chlor-phenyl)-1,2-dibromethyl)-2,2-dimethyl-cyclopropan-1-(R,S)-carbonsäure-α-(R,S)-cyano-3-phenoxy-4-fluor-benzylester als sehr zähes Öl.

Die Struktur wird durch das $^1H$-NMR-Spektrum bestätigt.

$^1H$-NMR-Spektrum (in $CDCl_3$/TMS):

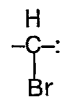: 5,3–5,85 τ (m/l H).

Beispiel 2:

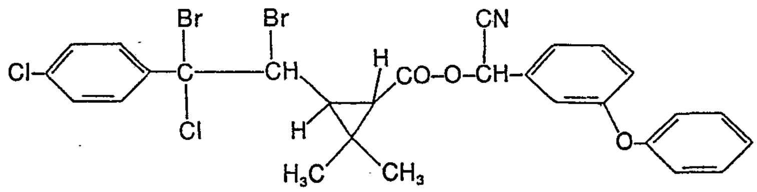

2,25 g (0,01 Mol) 3-Phenoxy-α-cyanbenzylalkohol und 4,63 g (0,01 Mol) trans-3-(2-Chlor-2-(4-chlor-phenyl)-1,2-dibromethyl)-2,2-dimethyl-cyclopropan-1-(R,S)-carbonsäurechlorid werden in 100 ml wasserfreiem Toluol gelöst und bei 25–30 °C 0,8 g (0,01 Mol) Pyridin, gelöst in 200 ml Toluol, unter Rühren zugetropft. Anschliessend wird weitere 3 Stunden bei 25 °C gerührt. Das Reaktionsgemisch wird in 150 ml Wasser gegossen, die organische Phase abgetrennt und nochmals mit 100 ml Wasser gewaschen. Anschliessend wird die Toluolphase über Natriumsulfat getrocknet und das Lösungsmittel im Wasserstrahlvakuum abdestilliert. Letzte Lösungsmittelreste werden durch kurzes Andestillieren bei 60 °C/l Torr Badtemperatur entfernt. Man erhält 6 g (92% der Theorie) trans-3-(2-Chlor-2-(4-chlor-phenyl)-1,2-dibromethyl)-2,2-dimethyl-cyclopropan-1-(R,S)-carbonsäure-α-(R,S)-cyano-3-phenoxy-benzylester als zähes Öl. Die Struktur wird durch das $^1H$-NMR-Spektrum bestätigt.

$^1H$-NMR-Spektrum (in $CDCl_3$/TMS):

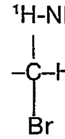: 5,2–5,9 τ (m/l H).

Analog Beispiel 1 oder 2 können die nachstehend aufgeführten Verbindungen hergestellt werden:

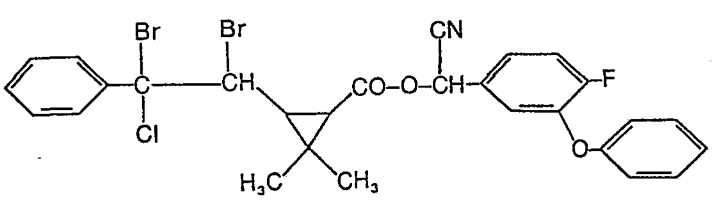

$^1H$-NMR-Spektrum (in $CDCl_3$/TMS)

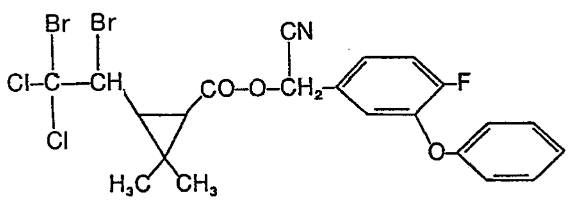

5,45–5,85 τ (m/1 H) = 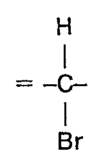

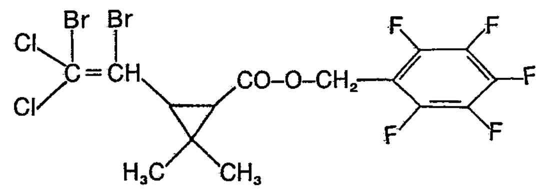

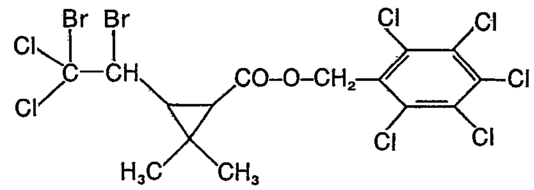

5,4–5,85 τ
(m/1 H)

= 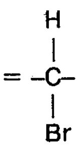

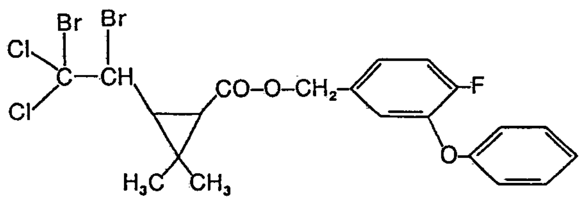

Brechungsindex:
$n_D^{20}$: 1,5717

Beispiele zur Herstellung von Ausgangsverbindungen:

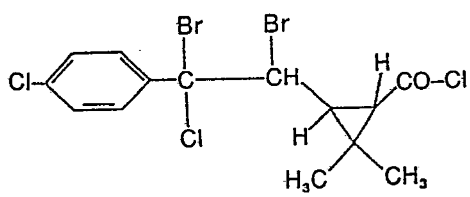

14 g trans-3-(2-Chlor-2-(4-chlor-phenyl)-1,2-dibromethyl)-2,2-dimethyl-cyclopropan-1-(R,S)-carbonsäure werden in 100 ml Tetrachlorkohlenstoff gelöst, mit 75 g Thionylchlorid versetzt und 4 Stunden zum Rückfluss erhitzt. Anschliessend wird das Lösungsmittel und überschüssiges Thionylchlorid im Vakuum abgezogen. Man erhält 13,7 g (77,8% der Theorie) trans-3-(2-Chlor-2-(4-chlor-phenyl)-1,2-dibromethyl)-2,2-dimethyl-cyclopropan-1-(R,S)-carbonsäurechlorid als zähes Öl.

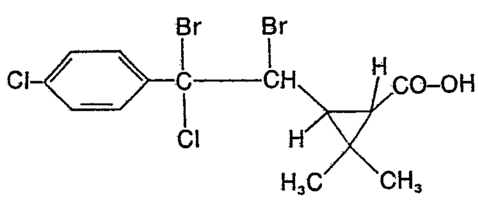

14 g (0,0492 Mol) trans-3-(E,Z-2-Chlor-2-(4-chlor-phenyl)-vinyl)-2,2-dimethyl-cyclopropan-1-(R,S)-carbonsäure werden in 90 ml Tetrachlorkohlenstoff gelöst und bei 20–25 °C 8 g (0,05 Mol) Brom, gelöst in 10 ml Tetrachlorkohlenstoff, langsam unter Rühren zugetropft. Anschliessend wird 1 Stunde bei Raumtemperatur nachgerührt, dann das Lösungsmittel im Vakuum abgezogen und der Rückstand mit 80 ml n-Hexan verrührt. Der entstandene kristalline Feststoff wird abgesaugt. Man erhält 15 g (68,6% der Theorie) trans-3-(2-Chlor-2-(4-chlor-phenyl)-1,2 - dibromethyl) - 2,2-dimethyl-cyclopropan-1-(R,S)-carbonsäure als hellgelben Feststoff mit dem Schmelzpunkt 150 °C.

Analog lässt sich darstellen:

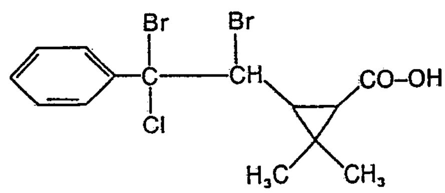

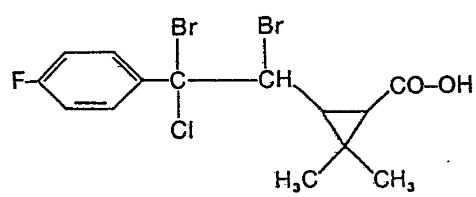

Die Wirkstoffe eignen sich bei guter Pflanzenverträglichkeit und günstiger Warmblütertoxizität zur Bekämpfung von tierischen Schädlingen, insbesondere Insekten, Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularrits, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon chochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemässen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,001 und 1 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemässen Wirkstoffe eignen sich auch zur Bekämpfung von Ekto- und Endoparasiten auf dem veterinärmedizinischen Gebiet.

Die Anwendung der erfindungsgemässen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgiessens (pour-on and spot-on) und des Einpuderns sowie durch parenterale Anwendung in Form beispielsweise der Injektion.

Beispiel

Phaedon-Larven-Test

Lösungsmittel: 3 Gewichtsteile Aceton

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, dass alle Käfer-Larven abgetötet wurden; 0% bedeutet, dass keine Käfer-Larven abgetötet wurden.

Bei diesem Test zeigen z.B. die folgenden Verbindungen der Herstellungsbeispiele überlegene Wirksamkeit gegenüber dem Stand der Technik: 1 und 2.

Beispiel

Test mit Stomoxys calcitrans

Lösungsmittel:

35 Gewichtsteile Äthylenglykolmonomethyläther

35 Gewichtsteile Nonylphenolpolyglykoläther

Zur Herstellung einer zweckmässigen Wirkstoffzubereitung vermischt man drei Gewichtsteile Wirkstoff mit sieben Gewichtsteilen des oben angegebenen Lösungsmittelgemisches und verdünnt das so erhaltene Konzentrat mit Wasser auf die jeweils gewünschte Konzentration.

10 adulte Stomoxys calcitrans werden in Petrischalen gebracht, die Filterpapierscheiben entsprechender Grösse enthalten, die einen Tag vor Versuchsbeginn mit 1 ml der zu testenden Wirkstoffzubereitung durchtränkt wurden. Nach 3 Stunden wird der Abtötungsgrad bestimmt.

Bei diesem Test zeigen z.B. die folgenden Verbindungen der Herstellungsbeispiele überlegene Wirkung gegenüber dem Stand der Technik: 1 und 2.

Beispiel

Test mit Boophilus microplus resistent

Lösungsmittel:

35 Gewichtsteile Äthylenglykolmonomethyläther

35 Gewichtsteile Nonylphenolpolyglykoläther

Zur Herstellung einer zweckmässigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 1 und 2.

Beispiel
Test mit Musca autumnalis
Lösungsmittel:
35 Gewichtsteile Äthylenglykolmonomethyläther
35 Gewichtsteile Nonylphenolpolyglykoläther

Zur Herstellung einer zweckmässigen Wirkstoffzubereitung vermischt man drei Gewichtsteile Wirkstoff mit sieben Gewichtsteilen des oben angegebenen Lösungsmittelgemisches und verdünnt das so erhaltene Konzentrat mit Wasser auf die jeweils gewünschte Konzentration.

10 adulte Musca autumnalis werden in Petrischalen gebracht, die Filterpapierscheiben entsprechender Grösse enthalten, die einen Tag vor Versuchsbeginn mit 1 ml der zu testenden Wirkstoffzubereitung durchtränkt wurden. Nach 3 Stunden wird der Abtötungsgrad bestimmt.

Bei diesem Test zeigen z.B. die folgenden Verbindungen der Herstellungsbeispiele überlegene Wirkung gegenüber dem Stand der Technik: 1 und 2.

Beispiel
Test mit Lucilia cuprina res.-Larven
Emulgator:
35 Gewichtsteile Äthylenglykolmonomethyläther
35 Gewichtsteile Nonylphenolpolyglykoläther

Zur Herstellung einer zweckmässigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 1 und 2.

Beispiel
Grenzkonzentrations-Test / Bodeninsekten
Testinsekt: Agrotis Segetum-Larven (im Boden)
Lösungsmittel: 3 Gewichtsteile Aceton
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und lässt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z.B. die folgenden Verbindungen der Herstellungsbeispiele übelegene Wirkung gegenüber dem Stand der Technik: 2.

**Patentansprüche**

1. Substituierte 3-(1,2-Dibrom-alkyl)-2,2-dimethyl-cyclopropan-1-carbonsäureester der Formel I

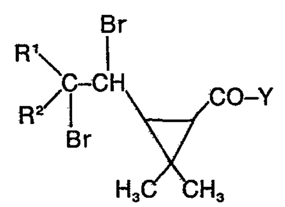

(I)

in welcher

$R^1$ für Halogen oder für gegebenenfalls substituiertes Phenyl oder Alkyl steht,

$R^2$ für Wasserstoff, Halogen oder gegebenenfalls substituiertes Alkyl steht und

Y für den Rest

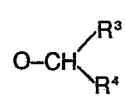

steht, wobei

$R^3$ für Wasserstoff, Cyano oder für gegebenenfalls substituiertes Alkyl, Alkenyl oder Alkinyl steht, und

$R^4$ für Phenyl steht, welches durch Halogen und/oder durch gegebenenfalls durch Halogen, Alkyl oder Halogenalkyl substituiertes Phenoxy substituiert ist mit der Massgabe, dass der Phenylrest wenigstens einen Halogen-Substituenten enthält, wenn $R^1$ für Methyl oder Halogen steht.

2. Verfahren zur Herstellung von Verbindungen der Formel (I)

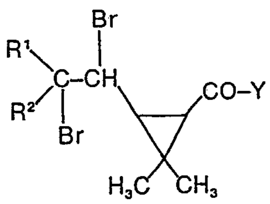

(I)

in welcher

$R^1$ für Halogen oder für gegebenenfalls substituiertes Phenyl oder Alkyl steht,

$R^1$ für Wasserstoff, Halogen oder gegebenenfalls substituiertes Alkyl steht und

Y für den Rest

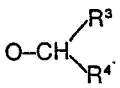

steht, wobei

$R^3$ für Wasserstoff, Cyano oder für gegebenenfalls substituiertes Alkyl, Alkenyl oder Alkinyl steht, und

$R^4$ für Phenyl steht, welches durch Halogen und/oder durch gegebenenfalls durch Halogen, Alkyl oder Halogenalkyl substituiertes Phenoxy substituiert ist mit der Massgabe, dass der Phenylrest wenigstens einen Halogen-Substituenten enthält, wenn $R^1$ für Methyl oder Halogen steht,

dadurch gekennzeichnet, dass man

a) 3-Alkenyl-2,2-dimethyl-cyclopropan-1-carbonsäureester der Formel (II)

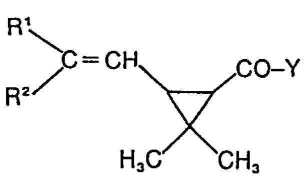

(II)

in welcher

$R^1$, $R^2$ und Y die oben angegebenen Bedeutungen haben, mit Brom, gegebenenfalls in Gegenwart eines Verdünnungsmittels umsetzt, oder

b) 3-(1,2-Dibrom-alkyl)-2,2-dimethyl-cyclopropan-1-carbonsäuren der Formel III

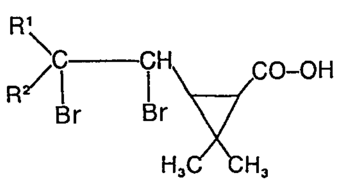

(III)

in welcher

$R^1$ und $R^2$ die oben angegebenen Bedeutungen haben, oder reaktionsfähige Derivate derselben mit einem bei Pyrethroiden üblichen Alkohol der Formel IV

Y–H (IV)

in welcher

Y die oben angegebene Bedeutung hat, oder mit reaktionsfähigen Derivaten desselben, gegebenenfalls in Gegenwart von Säurebindemitteln, gegebenenfalls in Gegenwart von Katalysatoren und gegebenenfalls in Gegenwart von Verdünnungsmitteln umsetzt.

3. 3-(1,2-Dibrom-alkyl)-2,2-dimethyl-cyclopropan-1-carbonsäure-derivate der Formel III a

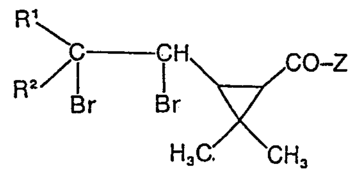

(III a)

in welcher

$R^2$ für Wasserstoff, Halogen oder gegebenenfalls substituiertes Alkyl steht,

$R^1$ für gegebenenfalls substituiertes Phenyl steht und

Z für Hydroxy, Chlor, $C_1$–$C_4$-Alkoxy oder für $O^{\ominus}M^{\oplus}$ steht, wobei $M^{\oplus}$ für ein Ammoniumion oder ein Alkali- oder Erdalkalimetallionenäquivalent steht.

4. Verfahren zur Herstellung von Verbindungen der Formel (III a), dadurch gekennzeichnet, dass man 3-Alkenyl-2,2-dimethyl-cyclopropan-1-carbonsäuren der Formel V

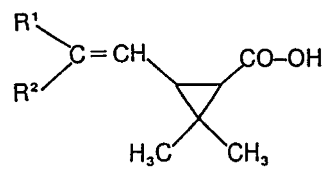

(V)

in welcher

$R^1$ und $R^2$ die oben angegebenen Bedeutungen haben, mit Brom, gegebenenfalls in Gegenwart eines Verdünnungsmittels umsetzt und die Bromierungsprodukte gegebenenfalls nach üblichen Methoden in die entsprechenden Carbonsäurederivate nach Formel (III a) umwandelt.

5. Schädlingsbekämpfungsmittel, gekennzeichnet durch einen Gehalt an mindestens einem substituierten 3-(1,2-Dibrom-alkyl)-2,2-dimethyl-cyclopropan-1-carbonsäureester der Formel (I) gemäss Anspruch 1.

6. Verwendung von substituierten 3-(1,2-Dibrom-alkyl)-2,2-dimethyl-cyclopropan-1-carbonsäure-estern der Formel (I) gemäss Anspruch 1 zur Bekämpfung von Schädlingen.

7. Verfahren zur Herstellung von Schädlingsbekämpfungsmitteln, dadurch gekennzeichnet, dass man substituierte 3-(1,2-Dibrom-alkyl)-2,2-dimethyl-cyclopropan-1-carbonsäureester der Formel (I) gemäss Anspruch 1 mit Streckmitteln und/oder oberflächenaktiven Mitteln vermischt.

8. Trans-3-(2-Chlor-2-(4-chlorphenyl)-1,2-dibromethyl)-2,2-dimethyl-cyclopropan-1-(R,S)-carbonsäure(R,S)-cyano-3-phenoxy-4-fluor-benzylester der Formel

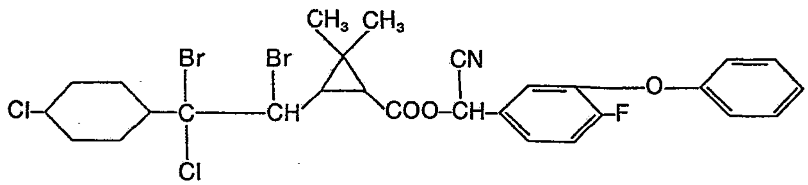

**Claims**

1. Substituted 3-(1,2-dibromo-alkyl)-2,2-dimethyl-cyclopropane-1-carboxylic acid esters for the formula I

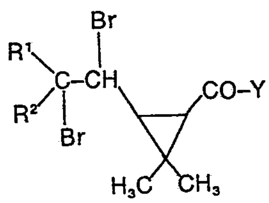

(I)

in which

$R^1$ represents halogen or optionally substituted phenyl or alkyl,

$R^2$ represents hydrogen, halogen or optionally substituted alkyl and

Y represents the radical

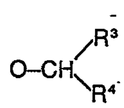

wherein

$R^3$ represents hydrogen, cyano or optionally substituted alkyl, alkenyl or alkinyl, and

$R^4$ represents phenyl which is substituted by halogen and/or by phenoxy which is optionally substituted by halogen, alkyl or halogenoalkyl, with the proviso that the phenyl radical contains at least one halogen substituent if $R^1$ represents methyl or halogen.

2. Process for the preparation of compounds of the formula (I)

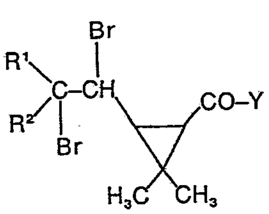

(I)

in which

$R^1$ represents halogen or optionally substituted phenyl or alkyl,

$R^1$ represents hydrogen, halogen or optionally substituted alkyl and

Y represents the radical

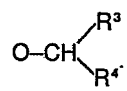

wherein

$R^3$ represents hydrogen, cyano or optionally substituted alkyl, alkenyl or alkinyl, and

$R^4$ represents phenyl which is substituted by halogen and/or by phenoxy which is optionally substituted by halogen, alkyl or halogenoalkyl, with the proviso that the phenyl radical contains at least one halogen substituent if $R^1$ represents methyl or halogen,

characterised in that

a) 3-alkenyl-2,2-dimethyl-cyclopropane-1-carboxylic acid esters of the formula (II)

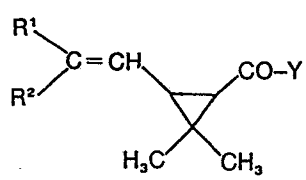

(II)

in which

$R^1$, $R^2$ and Y have the meanings indicated above, are reacted with bromine, if appropriate in the presence of a diluent, or

b) 3-(1,2-dibromo-alkyl)-2,2-dimethyl-cyclopropane-1-carboxylic acids of the formula III

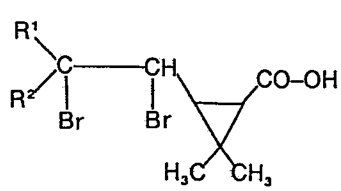

(III)

in which

$R^1$ and $R^2$ have the meanings indicated above, or reactive derivatives thereof, are reacted with an alcohol which is customary in pyrethroids, of the formula IV

Y–H (IV)

in which

Y has the meaning indicated above,

or with reactive derivatives thereof, if appropriate in the presence of acid-binding agents, if appropriate in the presence of catalysts and if appropriate in the presence of diluents.

3. 3-(1,2-Dibromo-alkyl)-2,2-dimethyl-cyclopropane-1-carboxylic acid derivatives of the formula III a

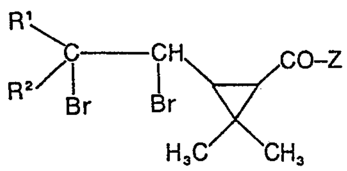

(III a)

in which

$R^2$ represents hydrogen, halogen or optionally substituted alkyl,

$R^1$ represents optionally substituted phenyl and

Z represents hydroxyl, chlorine, $C_1$–$C_4$-alkoxy or $O^{\ominus}M^{\oplus}$, wherein

$M^{\oplus}$ represents an ammonium ion or one alkali metal or alkaline earth metal ion equivalent.

4. Process for the preparation of compounds of the formula (III a), characterised in that 3-alkenyl-2,2-dimethyl-cyclopropane-1-carboxylic acids of the formula V

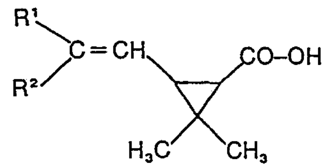

(V)

in which

$R^1$ and $R^2$ have the meanings indicated above, are reacted with bromine, if appropriate in the presence of a diluent, and the bromination products are optionally converted, by customary methods, into the corresponding carboxylic acid derivatives according to formula (III a).

5. Agents for combating pests, characterised in that they contain at least one substituted 3-(1,2-dibromo-alkyl)-2,2-dimethyl-cyclopropane-1-carboxylic acid ester of the formula (I) according to Claim 1.

6. Use of substituted 3-(1,2-dibromo-alkyl)-2,2-dimethyl-cyclopropane-carboxylic acid esters of the formula (I) according to Claim 1 for combating pests.

7. Process for the preparation of agents for combating pests, characterised in that substituted 3-(1,2-dibromo-alkyl)-2,2-dimethyl-cyclopropane-1-carboxylic acid esters of the formula (I) according to Claim 1 are mixed with extenders and/or surface-active agents.

8. Trans-3-(2-chloro-2-(4-chlorophenyl)-1,2-dibromoethyl)-2,2-dimethyl-cyclopropane-1-(R,S)-carboxylic acid -(R,S)-cyano-2-phenoxy-4-fluorobenzyl ester of the formula

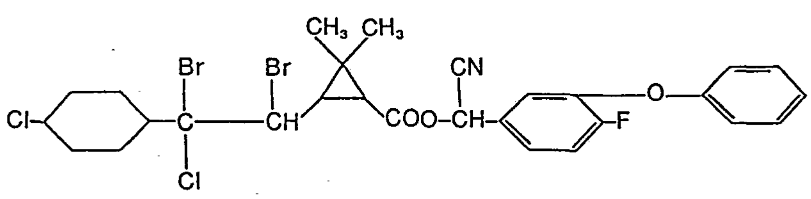

**Revendications**

1. Esters d'acides 3-(1,2-dibromo-alkyl)-2,2-diméthyl-cyclopropane-1-carboxyliques substitués de formule I:

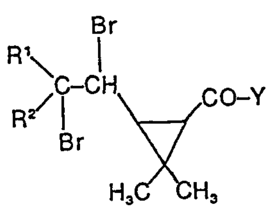

(I)

dans laquelle

$R^1$ représente un atome d'halogène ou un groupe alkyle ou un groupe phényle éventuellement substitué,

$R^2$ représente un atome d'hydrogène, un atome d'halogène ou un groupe alkyle éventuellement substitué, et

Y représente le radical

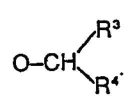

où

$R^3$ représente un atome d'hydrogène, un groupe cyano ou un groupe alcynyle, un groupe alcényle ou un groupe alkyle éventuellement substitué, et

$R^4$ représente un groupe phényle qui est substitué par un atome d'halogène et/ou par un groupe phénoxy éventuellement substitué par un atome d'halogène, par un groupe alkyle ou par un

groupe halogénalkyle, avec cette réserve que le groupe phényle contient au moins un substituant halogéné lorsque $R^1$ représente un groupe méthyle ou un atome d'halogène.

2. Procédé de préparation de composés de formule (I):

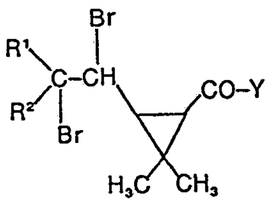

(I)

dans laquelle

$R^1$ représente un atome d'halogène ou un groupe alkyle ou un groupe phényle éventuellement substitué,

$R^2$ représente un atome d'hydrogène, un atome d'halogène, ou un groupe alkyle éventuellement substitué, et

Y représente le radical

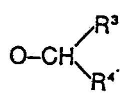

où

$R^3$ représente un atome d'hydrogène, un groupe cyano ou un groupe alcynyle, un groupe alcényle ou un groupe alkyle éventuellement substitué, et

$R^4$ représente un groupe phényle qui est substitué par un atome d'halogène et/ou par un groupe phénoxy éventuellement substitué par un atome d'halogène, par un groupe alkyle ou par un groupe halogénalkyle, avec cette réserve que le groupe phényle contient au moins un substituant halogéné lorsque $R^1$ représente un groupe méthyle ou un atome d'halogène,

caractérisé en ce que:

a) on fait réagir des esters d'acides 3-alcényl-2,2-diméthyl-cyclopropane-1-carboxyliques de formule (II):

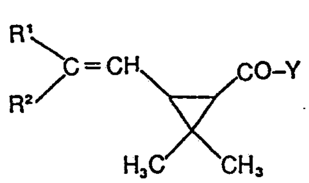

(II)

dans laquelle

$R^1$, $R^2$ et Y ont les significations indiquées ci-dessus, avec du brome, éventuellement en présence d'un diluant, ou

b) on fait réagir des acides 3-(1,2-dibromo-alkyl)-2,2-diméthyl-cyclopropane-1-carboxyliques de formule III:

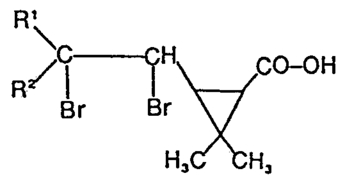

(III)

dans laquelle

$R^1$ et $R^2$ ont les significations indiquées ci-dessus, ou des dérivés réactifs de ces acides, avec un alcool habituellement utilisé dans les pyréthroïdes et répondant à la formule IV:

$$Y\text{–}H \qquad (IV)$$

dans laquelle

Y a la signification indiquée ci-dessus,

ou avec des dérivés réactifs de cet alcool, éventuellement en présence d'agents fixateurs d'acides, éventuellement en présence de catalyseurs et éventuellement en présence de diluants.

3. Dérivés d'acides 3-(1,2-dibromo-alkyl)-2,2-diméthyl-cyclopropane-1-carboxyliques de formule IIIa:

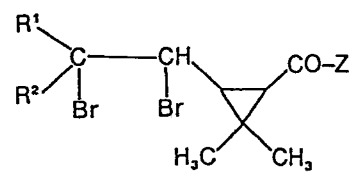

(III a)

dans laquelle

$R^2$ représente un atome d'hydrogène, un atome d'halogène ou un groupe alkyle éventuellement substitué,

$R^1$ représente un groupe phényle éventuellement substitué, et

Z représente un groupe hydroxy, un atome de chlore, un groupe alcoxy en $C_1$–$C_4$ ou $O^{\ominus}M^{\oplus}$, $M^{\oplus}$ représentant un ion ammonium ou un équivalent d'un ion de métal alcalin ou alcalino-terreux.

4. Procédé de préparation de composés de formule (IIIa), caractérisé en ce qu'on fait réagir des acides 3-alcényl-2,2-diméthyl-cyclopropane-1-carboxyliques de formule V:

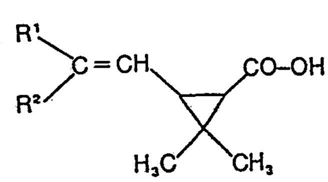

(V)

dans laquelle

$R^1$ et $R^2$ ont les significations indiquées ci-dessus, avec du brome, éventuellement en présence d'un diluant et on transforme éventuellement les produits de bromation en dévirés d'acides carboxyliques correspondants suivant la formule (IIIa) selon des méthodes habituelles.

5. Parasiticides, caractérisés en ce qu'ils contiennent au moins un ester d'acide 3-(1,2-dibromo-alkyl)-2,2-diméthyl-cyclopropane-1-carboxylique substitué de formule (I) suivant la revendication 1.

6. Utilisation d'esters d'acides 3-(1,2-dibromo-alkyl)-2,3-diméthyl-cyclopropane-1-carboxyliques substitués de formule (I) suivant la revendication 1 pour combattre les parasites.

7. Procédé de préparation de parasiticides caractérisé en ce qu'on mélange des esters d'acides 3-(1,2-dibromo-alkyl)-2,2-diméthyl-cyclopropane-1-carboxyliques substitués de formule (I) suivant la revendication 1 avec des diluants et/ou des agents tensio-actifs.

8. L'ester α-(R,S)-cyano-3-phénoxy-4-fluorobenzylique de l'acide trans-3-(2-chloro-2-(4-chlorophényl)-1,2-dibrométhyl)-2,2-diméthyl-cyclopropane-1-(R,S)-carboxylique de formule: